# FASCICULE DE BREVET EUROPEEN

(11) **EP 1 786 405 B1**
(45) Date de publication et mention de la délivrance du brevet: **22.02.2012**
(21) Numéro de dépôt: 05793300.4
(22) Date de dépôt: 22.07.2005
(51) Int. Cl.: A61P 25/32, A61K 9/51, A61K 9/107, A61K 9/127

(54) **Utilisations et composés pour percevoir ou contrôler la prise d'alcool**
Verwendung und Substanzen zur Wahrnehmung oder Kontrolle von Alkoholeinnahme
Use and compounds for perceiving or controlling alcool intake

(30) Priorité: 23.07.2004 FR 0408155
(43) Date de publication de la demande: 23.05.2007
(60) Demande divisionnaire: 10178121.9
(73) Titulaire: JNC Innovations, 75 008 Paris (FR)
(72) Inventeur: Colombani Jean-Noël, 75116 Paris (FR)
(74) Mandataire: L'Helgoualch, Jean
(86) Numéro de dépôt international: PCT/FR2005/001903
(87) Numéro de publication internationale: WO 2006/018521

(56) Documents cités:
- EP-A- 0 201 134
- WO-A-93/24106
- WO-A-99/36053
- FR-A- 2 642 305
- US-A- 5 744 158
- US-A- 5 908 697
- KHANBABAEE K ET AL: "Tannins: classification and definition." NATURAL PRODUCT REPORTS. DEC 2001, vol. 18, no. 6, décembre 2001 (2001-12), pages 641-649, XP002322015 ISSN: 0265-0568

## Description

La présente demande décrit des compositions et méthodes permettant de percevoir la prise d'alcool chez des sujets. Elle décrit notamment des composés assurant une libération in vivo de substances actives, cette libération étant conditionnée par la présence et/ou la teneur en alcool chez un sujet. L'invention concerne l'objet des revendications 1 et 2.

Des aspects préférés de l'invention sont décrit dans les revendications 3-11.

Il existe environ cinq millions de buveurs excessifs en France et cinq cent millions dans le monde. Non dépendants à l'alcool, dans la mesure où ils peuvent s'arrêter de boire pendant des périodes de quelques jours à plusieurs semaines, par exemple, ils présentent toutefois la caractéristique qu'une fois la molécule d'alcool introduite dans la bouche, ils ne peuvent plus arrêter ou contrôler leur consommation. L'expérience acquise par le demandeur, tant sur le plan hospitalier qu'en suivi libéral, montre que ces patients sont très demandeurs d'une aide à l'arrêt de cette consommation abusive et incontrôlée et qu'ils sont psychologiquement décidés à prévenir cette consommation avant que ne soit réalisé le contact avec la molécule d'alcool.

D'autre part, certains patients peuvent, par exemple en raison d'une pathologie et/ou d'une intervention chirurgicale, nécessiter une abstinence momentanée ou de longue durée en alcool. Il peut s'agir notamment de patients présentant des pathologies nécessitant une abstinence pour des raisons nutritionnelle, cardiovasculaire, diabétique, hépatique, neurologique, ophtalmologique, vasculaire (hypertension), etc. L'abstinence peut également résulter de causes iatrogènes (contre-indication d'association avec la présence d'autres molécules thérapeutiques déjà prises par le patient).

Par ailleurs, certaines personnes présentent une hyper-réactivité à la prise d'alcool et peuvent souhaiter en prévenir les effets dans leurs relations sociales, personnelles ou professionnelles.

Enfin, dans le cadre des actions de prévention routière, il pourrait être utile de disposer d'alertes permettant de ne pas dépasser le dosage légal, fournissant ainsi une réponse quasi-ludique à la maîtrise de la sécurité.

Les molécules actuellement disponibles pour la lutte contre la consommation d'alcool sont essentiellement de deux sortes:
L'ESPERAL à base de disulfirame, molécule à visée anta-buse, qui rend impossible toute consommation d'alcool après prise du médicament, sous peine de générer de nombreux troubles physiques: Nausées, vomissements, douleurs digestives, palpitations, tachycardie, etc. C'est cet effet secondaire qui est recherché mais il peut malheureusement entraîner des troubles pouvant devenir majeurs et entraîner un risque vital, ce qui conduit à limiter grandement sa prescription.

Le REVIAL (naltrexone) et l'AOTAL (acamprosate), molécules qui, à l'inverse de l'ESPERAL, n'entraînent pas d'effet secondaire en cas de consommation d'alcool. Leur mode d'action est plus complexe, puisqu'il vise à diminuer progressivement l'impulsion vers la consommation exagérée d'alcool par des moyens bio-endocriniens dont la bonne efficacité reste encore controversée.

Dans ces conditions, il existe un vide médical important, et un besoin très grand de solutions ou traitements permettant aux sujets de contrôler leur prise d'alcool, de manière ciblée, temporaire et volontaire.

La présente invention permet de remédier à ce besoin. La présente invention fournit en effet une utilisation permettant une maîtrise de l'absorption d'alcool dans une démarche volontaire des utilisateurs.

La présente demande repose sur la mise au point de composés particuliers, provoquant chez le sujet un rejet de l'alcool. Les composés décrits dans la présente demande combinent avantageusement une substance dénaturante présentant des propriétés astringente et/ou acide et une membrane assurant une libération contrôlée et locale. Ces composés peuvent être dosés et/ ou formulés de différentes façons, pour moduler l'effet produit et sa durée. Ainsi par exemple, les composés peuvent être préparés pour agir lorsque la concentration d'alcool au contact des composés atteint ou dépasse un certain seuil. De même, les composés peuvent être préparés ou formulés pour agir pendant une période de temps déterminée, par exemple de 1 à 4 heures. Ainsi, différents modes de mise en oeuvre sont possibles, assurant un domaine d'application très large des molécules.

Plus particulièrement, un premier aspect décrit un composé biocompatible, caractérisé en ce qu'il comprend une substance dénaturant l'alcool enrobée dans une membrane non-hydrosoluble mais soluble dans l'alcool. L'invention repose ainsi sur la mise au point de composés complexes, assurant la libération de substances dénaturant l'alcool, et donc produisant une réaction de rejet de l'alcool, cette libération étant conditionnée par la présence d'alcool (ou la teneur en alcool) chez le sujet. Les composés assurent avantageusement un effet dénaturant sur le goût en présence de la molécule d'éthanol (C₂HₛOH). Cette molécule étant présente dans tous les alcools, ceci confère aux composés une application générale, pour tous types d'alcools, dans un contexte curatif ou préventif. Les composés n'ont pas pour objectif de transformer les molécules d'alcool ou leur réactivité, mais de libérer une substance dénaturante de l'alcool, créant ainsi un effet répulsif et/ou révulsif.

Au sens de la présente invention, on entend par le terme "substance" aussi bien une molécule unique, par exemple isolée, qu'un mélange de constituants divers. On entend également par le terme "composé biocompatible" tout composé compatible avec un usage chez l'homme, c'est-à-dire typiquement ne produisant pas d'effet toxique significatif.

Plus préférentiellement, les composés permettent une libération contrôlée et locale, par exemple dans la bouche d'un sujet, d'une substance alliant des propriétés répulsive et révulsive. La substance présente des propriétés astringente et acide.

Le goût se définit de manière générale par quatre caractéristiques : L'acidité, l'amertume l'astringence et le sucré, auxquels on ajoute l'unami. L'unami, identifié récemment, est une saveur qui correspond au monoglutamate de sodium. Par ailleurs, l'olfaction est responsable de 80% du goût par le système de la retro-olfaction. En outre, le goût inclut une infinité de saveurs, et c'est l'association odeurs / goût qui mobilise, chez le mammifère, l'ensemble des récepteurs des papilles linguales et du palais.

L'astringence, ou astriction, est une saveur de base qui est utilisée dans la classification des plantes. C'est une des treize saveurs primaires. L'astringence est une sensation qui se reconnaît mais qui est difficile à définir étant donné qu'elle est très rarement pure. Mêlée la plupart du temps dans les produits naturels à des sensations d'acidité et d'amertume qui viennent la renforcer et qui se confondent avec elle, l'astringence donne un impression de sécheresse et de rugosité, au lieu de l'humidité lisse habituellement perçue. L'astringence produit une impression de dureté et de piquant dans la bouche, accompagnée d'une inhibition de la salivation. L'astringence n'est pas due à des transformations biochimiques.

La substance utilisée dans l'invention possède des propriétés astringentes, c'est-à-dire cause, lorsqu'elle est libérée, une sensation d'astringence chez le sujet.

Il existe différents agents astringents d'origine végétale, comestibles. Il s'agit essentiellement de tannins, de la famille des polyphénols. Ceux-ci proviennent typiquement des pépins, cosses, enveloppes de fruits, coques, artichauts, cardes, fèves vertes, pommes à cidre, variétés tardives de pêches. En faibles quantités, les tannins améliorent la palatabilité des éléments. En revanche, en grandes quantités, ils conduisent à diminuer la prise alimentaire.

Dans un mode plus particulier de réalisation des composés, la substance enrobée comprend donc un tanin, de préférence de la famille des polyphénols. Il existe deux groupes de tanins de la famille des polyphénols, à savoir les tanins condensés et les tanins hydrosolubles. Les tanins condensés, appartenant généralement à la famille des flavonoïdes, sont typiquement des composés en C6-C3-C6. Dans le contexte de la présente invention, on préfère tout particulièrement les tanins condensés de la famille de flavonoïdes, notamment les tanins condensés, existant à l'état naturel, et utilisés en tant que tanins industriels.

Dans un autre mode particulier de réalisation des composés, la substance enrobée comprend un acide compatible avec un usage alimentaire, causant une sensation locale d'acidité chez le sujet. L'acide compatible utilisé est avantageusement choisi parmi l'acide malique, tannique, citrique, acétique ou lactique.

Un exemple de composé qui est une substance dénaturant l'alcool est le benzoate de denatonium, ou benzoate de benzyldiéthyl-[(2,6-xylylcarbamoyl)méthyl)] ammonium (Bitrex®) qui est connu pour sa très forte amertume, et procure un goût amer très prononcé au produit au contact duquel il est placé. Le benzoate de denatonium est généralement disponible sous forme de poudre très peu soluble dans l'eau et soluble dans les alcools. Il est également décrit un extrait purifié de Quassia amara, agent d'amertume naturel, disponible dans le commerce (Quasselect®). Suivant une variante, la demande décrit le dichloro-anisole, des épices telles que des extraits de gingembre, de curry ou de safran, ou encore un extrait de clou de girofle, sous forme de poudre ou d'essence. Un composé peut être

par exemple une combinaison d'un tanin et d'un acide compatible, ou d'une molécule possédant les deux propriétés, par exemple l'acide tannique. L'utilisation des substances indiquées ci-dessus, seules ou en mélange, permet de provoquer un rejet ou une répulsion vis-à-vis de l'alcool chez des sujets, et permet ainsi un contrôle adapté de la prise d'alcool.

La substance enrobée peut en outre comprendre des agents ou ingrédients supplémentaires, tels que des lipides, protides, protéines, glucides, etc. Ces différents ingrédients et/ou excipients permettent de moduler par exemple la consistance, la stabilité, l'efficacité et/ou le goût des composés. La composition finale des composés peut être ajustée par l'homme du métier, selon l'effet recherché, le type de sujet considéré, etc.

Le benzoate de denatonium procure une très forte amertume et peut être avantageusement associé à un parfum acceptable sur plan alimentaire qui peut assécher la cavité buccale ou la cloison nasale pour tempérer l'amertume du benzoate de denatonium. On peut par exemple associer le benzoate de denatonium à du menthol.

Les composés sont simples à utiliser, n'induisent pas d'effets secondaires significatifs, et leur durée d'action peut être ajustée, en faisant varier la dose de substance et/ou la nature de la membrane. Ainsi, plus le taux de substance dénaturante (de tannin et/ou d'acide) est élevé, plus le composé est doté de propriétés répulsives et révulsives à l'alcool. En outre, les composés peuvent également exercer un effet répulsif à l'appétit. L'efficacité et les propriétés des composés peuvent être modulées en jouant par exemple sur la teneur en tanins de la substance libérée, qui peut varier dans une large proportion. De même, le taux d'astringence est proportionnel au tannin rencontré dans toutes les formules des compositions d'alcool, de sorte que l'effet sera plus prononcé avec des alcools forts, qu'avec des alcools légers.

Plusieurs variantes peuvent ainsi être réalisées, comme par exemple un composé proprement répulsif, à forte teneur en substance dénaturante, éventuellement sous forme de pilule, pour les personnes les plus sensibilisées à la prise d'alcool, et pour lesquelles une saveur trop neutre ne serait pas suffisante. S'agissant d'une prise volontaire par les sujets concernés, il convient avantageusement de préparer des composés dont le goût n'est pas systématiquement insupportable, mais qui puissent présenter une saveur déplaisante et surtout dénaturante au regard du goût de l'alcool.

Comme indiqué précédemment, la substance dénaturante est entourée d'une membrane, assurant une libération contrôlée. La membrane entourant la substance peut être composée de tout matériau biocompatible, non-hydrosoluble mais soluble dans l'alcool. Ainsi, en l'absence d'alcool, la substance dénaturante n'est pas libérée et les composés de l'invention sont essentiellement sans effet. En revanche, en présence d'alcool (ou lorsque la concentration en alcool atteint un certain seuil), la membrane se dissout et la substance est libérée, provoquant une action dénaturante, essentiellement locale (c'est-à-dire principalement dans la bouche). La membrane présente de préférence des propriétés telles que, dans sa mise en oeuvre pratique, le composé assure une compatibilité acide-base (PH) compatible avec un équilibre physiologique buccal et/ou nasal.

La membrane des composés peut être composée ou à base de différents matériaux, tels que notamment des lipides, polymères, composés biologiques, etc. Dans une variante particulière, la membrane est composée de particules, par exemple de nano-particules (e.g., comprenant la substance enrobée dans une paroi soluble dans l'alcool), dont la libération est déclenchée par le contact d'alcool. Les composés peuvent également être des micelles ou particules lipidiques stables dans l'eau, mais solubles dans l'alcool.

Les composés peuvent être sous différentes formes, telles que notamment sous forme liquide, solide de gel, crème, huile, pilule, etc. Ainsi, un autre aspect décrit concerne une composition comprenant un composé tel que défini précédemment et un excipient adapté à une application chez l'homme, de préférence une application locale, typiquement buccale ou nasale.

La composition peut ainsi comprendre différents diluants, excipients ou supports, tels que des solutions salines, physiologiques, aqueuses, tamponnées, des liants, des gélifiants, des particules, etc. La composition finale des composés peut être ajustée par l'homme du métier, selon l'effet recherché, le type de sujet considéré, etc. Dans un mode de mise en oeuvre particulier, les composés comprennent en outre un diluant, par exemple une solution aqueuse, pouvant comprendre en outre des lipides, protides, protéines, glucides et/ou tampons. Les composés et compositions sont typiquement destinés à une application locale, notamment orale ou nasale. Généralement, ils sont appliqués sur les muqueuses, selon toute forme galénique appropriée. Les saveurs de base (acide, amère, salée, sucrée) sont captées au niveau de la langue et de toute la bouche en des points sensibles particuliers à savoir, de l'avant au fond de la bouche :
- bout de la langue : sucré
- gencives et dessus de la langue : acide,
- périphérie de la langue : salé,
- fond de la langue: amer,
- bout de la langue : picotement,
- langue: astringence,
- palais: chaleur,
- partout: consistance.

Afin d'assurer une meilleure efficacité aux composés, une application buccale ou nasale est donc privilégiée, et toute formulation ou méthode permettant une telle application peut être employée dans le cadre de la présente invention.

Parmi les différentes formes galéniques existantes, on préfère tout particulièrement une forme galénique introduite dans la bouche, respectant les conditions physiologiques de celle-ci. On peut citer notamment des patchs, sprays, gels, crèmes, pilules, etc. On peut mentionner, à titre particulièrement intéressant:
- le patch buccal, dont une application est d'ores et déjà effective dans le traitement des aphtes buccaux au Japon,
- le spray buccal ou nasal, dont il existe aussi plusieurs applications ORL, dentaires, en infectiologie ou en anesthésiologie,
- les crèmes buccales ou nasales, qui permettent d'obtenir une épaisseur de membrane très faible et facilement supportable en tant que corps étranger pour un individu ne présentant aucun trouble à traiter,
- la petite feuillette appuyée à la voûte du palais, libérant la substance contenue dans les membranes et pouvant être accentuée ou pas par simple pression de la langue.

Un mode de réalisation particulièrement préféré de l'invention comprend l'utilisation d'un composé tel que défini ci-dessus sous forme de patch ou de spray buccal.

Le patch buccal comprenant la substance dénaturante selon la présente demande peut être avantageusement constitue par un support en une matière délitable dans l'eau et la salive, par exemple un tissu compatible, portant des microbilles enrobées comprenant la substance dénaturante selon l'invention. Plus particulièrement, le support peut être constitué d'un ruban comportant plusieurs zones successives prédécoupées, dont chacune porte le même nombre de microbilles et constitue ainsi un patch buccal que l'utilisateur peut aisément séparer de la bande et le place dans la bouche, par collage au voile du palais. L'enrobage des microbilles permet d'isoler la substance dénaturante et de ne la libérer qu'au contact d'un alcool.

Il est avantageux de préparer les microbilles du patch buccal par microencapsulation dans un milieu approprié puis enrobage au moyen d'une composition adaptée, résistant à l'eau mais soluble dans les alcools, comme par exemple la zéine, qui est un excipient couramment utilisé dans les compositions pharmaceutiques. L'enrobage a pour but de masquer l'amertume de la substance dénaturante et de libérer la substance dès que la concentration en alcool, par exemple en alcool éthylique, est supérieure ou égale à 5% d'alcool pur.

Le spray buccal peut être préparé par les techniques classiques en dispersant des nanoparticules de substance dénaturante dans un support approprié.

Afin de contrôler ou déterminer l'effet des composés, la dose de substance et/ou la nature de la membrane (ou du support) peuvent être adaptés. Ainsi, comme indiqué précédemment, plus la teneur en substance dénaturante est importante, plus les composés produisent un effet prononcé et durable. D'autre part, selon la nature de la membrane (ou du support), la substance peut être libérée en fonction de différentes concentrations en alcool. Dans ce contexte, le dosage-seuil de concentration de l'alcool est déterminé typiquement par rapport à la quantité d'alcool présente au contact des composés, c'est-à-dire en volume par litre (et non en concentration d'alcool dans le sang). La demi-vie des composés peut être ajustée par l'homme du métier, par exemple entre une durée minimum de deux heures, éventuellement renouvelable, éventuellement avec un effet progressif croissant, dose dépendante du volume d'alcool mis en présence de la molécule dans la bouche. La durée pendant laquelle l'effet répulsif vis-à-vis du goût de l'alcool continue à être perçu dépend également de la nature et de la teneur en substance dénaturante présente dans les composés.

Par exemple, plus particulièrement dans le cas d'un patch buccal, la substance dénaturante est microencapsulée pour former des microbilles qui sont ensuite enrobée, comme indiqué ci-dessus, et la dimension moyenne des microbilles est comprise entre 0,05 et 1 mm, de préférence entre 0,1 et 0,4 mm. Les microbilles sont dosées de telle sorte que la quantité totale de substance dénaturante, par exemple de benzoate de denatonium, soit comprise entre 2 et 5 mg par patch.

Un autre aspect décrit dans la présente demande réside dans un procédé de préparation d'un composé tel que défini précédemment, comprenant l'enrobage d'une substance telle que définie ci-dessus avec une membrane non hydrosoluble mais soluble dans l'alcool. L'enrobage peut être réalisé par toute technique connue de l'homme du métier, selon la nature de la membrane choisie.

L'invention vise également l'utilisation d'un composé ou d'une composition tels que définis précédemment, pour percevoir la prise d'alcool chez des sujets.

Un autre aspect décrit concerne une méthode de régulation de la prise d'alcool chez un sujet, comprenant l'application au sujet, de préférence par voie buccale ou nasale, d'un composé ou d'une composition tels que décrits précédemment, de sorte que la consommation ultérieure d'alcool ou d'une quantité prédéterminée d'alcool provoque une dénaturation du goût.

Un aspect supplémentaire décrit concerne une méthode de régulation de la prise d'alcool chez un sujet par dénaturation du goût, comprenant l'application au sujet, de préférence par voie buccale ou nasale, d'un composé ou d'une composition tels que décrits précédemment, de sorte que la consommation ultérieure d'alcool ou d'une quantité prédéterminée d'alcool provoque un effet répulsif et! ou révulsif.

La sensibilité de la méthode est naturellement fonction du titre d'alcoolémie. Cette méthode repose sur le support de la molécule, qui pourra être choisi entre les différentes formes sus-décrites, mais aussi et surtout sur les caractéristiques de cette molécule, de sa membrane, de l'indication même et de l'aide thérapeutique qu'elle représente pour les personnes concernées. Par ailleurs, la molécule est avantageusement déposée dans la cavité buccale de telle manière qu'elle puisse être dans un endroit localisé de celle-ci, extractible à tout moment et totalement assimilable par l'organisme. Enfin, les propriétés, les caractéristiques et les modalités d'application des composés et méthodes peuvent être modifiées et/ou adaptées au fur et à mesure des nécessités pour les patients, ainsi que des possibilités dues aux progrès technologiques.

L'invention est applicable à tout sujet mammifère, typiquement humain, notamment chez l'adulte, et offre pour la première fois une solution adaptée et commode au contrôle de l'absorption d'alcool dans une démarche volontaire des utilisateurs.

## Revendications

1. Utilisation d'un composé biocompatible comprenant une substance créant un effet répulsif et/ou révulsif de l'alcool enrobée dans une membrane non-hydrosoluble mais soluble dans l'alcool, ladite substance enrobée comprenant un acide compatible avec un usage alimentaire et/ou un tanin, ou d'une composition contenant un tel composé et un excipient adaptée à une application buccale ou nasale, ladite substance présentant des propriétés astringente et/ou acide, pour percevoir la prise d'alcool chez des sujets.

2. Composé biocompatible comprenant une substance créant un effet répulsif et/ou révulsif de l'alcool enrobée dans une membrane non-hydrosoluble mais soluble dans l'alcool, ladite substance enrobée comprenant un acide compatible avec un usage alimentaire, et/ou un tanin, ou composition contenant un tel composé et un excipient adaptée à une application buccale ou nasale, ladite substance présentant des propriétés astringente et/ou acide, pour l'utilisation dans le contrôle de la prise d'alcool chez des buveurs excessifs ou des patients présentant des pathologies nécessitant une abstinence.

3. Utilisation ou composé pour l'utilisation selon les revendications 1 ou 2, **caractérisé en ce que** l'acide compatible avec un usage alimentaire est choisi parmi l'acide malique, tannique, citrique, acétique ou lactique.

4. Utilisation ou composé pour l'utilisation selon les revendications 1 ou 2, **caractérisé en ce que** le tanin est un tanin de la famille des polyphénols.

5. Utilisation ou composé pour l'utilisation selon la revendication 4, **caractérisé en ce que** le tanin est choisi parmi les tanins condensés, notamment de la famille de flavonoïdes, et les tanins hydrosolubles, de préférence les tanins condensés.

6. Utilisation ou composé pour l'utilisation selon l'une quelconque des revendications précédentes, **caractérisé en ce que** la membrane est composée de nanoparticules dont la libération est déclenchée par le contact d'alcool.

7. Utilisation ou composé pour l'utilisation selon l'une quelconque des revendications précédentes, **caractérisé en ce que** le composé comprend en outre un diluant, par exemple une solution aqueuse, pouvant comprendre en outre des lipides, protides, protéines, glucides et/ou tampons.

8. Utilisation ou composé pour l'utilisation selon l'une quelconque des revendications précédentes, **caractérisé en ce que** le composé ou la composition est conditionné pour une application buccale ou nasale, de préférence sous forme de patch, spray ou crème.

9. Utilisation ou composé pour l'utilisation selon la revendication 8, caractérisé en ce la composition est constituée par un patch buccal comprenant un support en matière délitable dans l'eau et la salive, portant des microbilles enrobées comprenant la substance dénaturant l'alcool.

10. Utilisation ou composé pour l'utilisation selon la revendication 9, **caractérisé en ce que** la dimension moyenne des microbilles est comprise entre 0,05 et 1 mm.

11. Utilisation ou composé pour l'utilisation selon l'une quelconque des revendications 9 et 10, **caractérisé en ce que** les microbilles sont dosées de telle sorte que la quantité totale de substance dénaturante soit comprise entre 2 et 5 mg par composition.

## Claims

1. The use of a biocompatible compound comprising a substance generating a repulsive and/or revulsive effect toward alcohol, coated in a non-water-soluble but alcohol-soluble membrane, said coated substance comprising an acid compatible with food use and/or a tannin, or a composition containing such a compound and an excipient suitable for buccal or nasal application, said substance having astringent and/or acid properties, in order to detect alcohol intake in subjects.

2. A biocompatible compound comprising a substance generating a repulsive and/or revulsive effect toward alcohol, coated in a non-water-soluble but alcohol-soluble membrane, said coated substance comprising an acid compatible with food use and/or a tannin, or a composition containing such a compound and an excipient suitable for buccal or nasal application, said substance having astringent and/or acid properties, for use in alcohol intake monitoring of heavy drinkers or patients having pathologies requiring abstinence.

3. The use or the compound for the use according to claims 1 or 2, **characterized in that** the acid compatible with food use is selected from malic, tannic, citric, acetic or lactic acid.

4. The use or the compound for the use according to claims 1 or 2, **characterized in that** the tannin is a tannin from the family of polyphenols.

5. The use or the compound for the use according to claim 4, **characterized in that** the tannin is selected from condensed tannins, notably from the family of flavonoids, and from water-soluble tannins, preferably condensed tannins.

6. The use or the compound for the use according to any of the preceding claims, **characterized in that** the membrane consists of nanoparticles, the release of which is triggered by the contact with alcohol.

7. The use or the compound for the use according to any of the preceding claims, **characterized in that** the compound further comprises a diluent, for example an aqueous solution, which may further comprise lipids, protids, proteins, carbohydrates and/or buffers.

8. The use or the compound for the use according to any of the preceding claims, **characterized in that** the compound or the composition is conditioned for buccal or nasal application, preferably as a patch, spray or cream.

9. The use or the compound for the use according to claim 8, **characterized in that** the composition is formed with a buccal patch comprising a support in a material disintegrable in water and saliva, bearing coated microbeads comprising the alcohol denaturing substance.

10. The use or the compound for the use according to claim 9, **characterized in that** the average size of the microbeads is comprised between 0.05 and 1 mm.

11. The use or the compound for the use according to any of claims 9 and 10, **characterized in that** the microbeads are metered so that the total amount of denaturing substance is comprised between 2 and 5 mg per composition.

## Patentansprüche

1. Verwendung einer biologisch verträglichen Verbindung, die eine Substanz umfasst, die eine Alkohol abstoßende und/oder ableitende Wirkung hervorruft, die mit einer nicht wasserlöslichen, aber in Alkohol löslichen Membran überzogen ist, wobei die überzogene Substanz eine mit einer Ernährungsanwendung verträgliche Säure und/oder ein Tannin umfasst oder eine Zusammensetzung, die eine derartige Verbindung und einen Hilfsstoff enthält, die für eine Mund- oder nasale Anwendung geeignet ist, wobei die Substanz adstringierende und/oder saure Eigenschaften aufweist, um die Aufnahme von Alkohol bei Personen zu erkennen.

2. Biologisch verträgliche Verbindung, die eine Substanz umfasst, die eine Alkohol abstoßende und/oder ableitende Wirkung hervorruft, die mit einer nicht wasserlöslichen, aber in Alkohol löslichen Membran überzogen ist, wobei die überzogene Substanz eine mit einer Ernährungsanwendung verträgliche Säure und/oder ein Tannin umfasst, oder Zusammensetzung, die eine derartige Verbindung und einen Hilfsstoff enthält, die für eine Mund- oder nasale Anwendung geeignet ist, wobei die Substanz adstringierende und/oder saure Eigenschaften aufweist, zur Verwendung bei der Kontrolle der Aufnahme von Alkohol bei exzessiven Trinkern oder Patienten, die Erkrankungen aufweisen, für die eine Abstinenz notwendig ist.

3. Verwendung oder Verbindung für die Verwendung nach den Ansprüchen 1 oder 2, **dadurch gekennzeichnet, dass** die mit einer Ernährungsanwendung verträglich Säure aus der Apfel-, Gerb-, Citronen-, Essig- oder Milchsäure ausgewählt ist.

4. Verwendung oder Verbindung für die Verwendung nach den Ansprüchen 1 oder 2, **dadurch gekennzeichnet, dass** das Tannin ein Tannin der Familie der Polyphenole ist.

5. Verwendung oder Verbindung für die Verwendung nach Anspruch 4, **dadurch gekennzeichnet, dass** das Tannin aus den kondensierten Tanninen, insbesondere aus der Familie der Flavonoide, und den wasserlöslichen Tanninen, vorzugsweise den kondensierten Tanninen, ausgewählt ist.

6. Verwendung oder Verbindung für die Verwendung nach einem der vorangehenden Ansprüche, **dadurch gekennzeichnet, dass** sich die Membran aus Nanopartikeln zusammensetzt, deren Freisetzung durch den Alkoholkontakt ausgelöst wird.

7. Verwendung oder Verbindung für die Verwendung nach einem der vorangehenden Ansprüche, **dadurch gekennzeichnet, dass** die Verbindung weiterhin einen Verdünner umfasst, beispielsweise eine wässrige Lösung, die weiterhin Lipide, Protide, Proteine, Kohlenhydrate und/oder Puffer umfassen kann.

8. Verwendung oder Verbindung für die Verwendung nach einem der vorangehenden Ansprüche, **dadurch gekennzeichnet, dass** die Verbindung oder die Zusammensetzung für eine Mund- oder nasale Anwendung konditioniert ist, vorzugsweise in Form eines Patches, Sprays oder einer Creme.

9. Verwendung oder Verbindung für die Verwendung nach Anspruch 8, **dadurch gekennzeichnet, dass** die Zusammensetzung von einem Mundpatch gebildet wird, das einen Träger aus einem in Wasser und Speichel auflösbaren Stoff umfasst, der umhüllte Mikrokügelchen trägt, die die den Alkohol denaturierende Substanz umfassen.

10. Verwendung oder Verbindung für die Verwendung nach Anspruch 9, **dadurch gekennzeichnet, dass** die durchschnittliche Größe der Mikrokügelchen zwischen 0,05 und 1 mm inklusive ist.

11. Verwendung oder Verbindung für die Verwendung nach einem der Ansprüche 9 und 10, **dadurch gekennzeichnet, dass** die Mikrokügelchen derart dosiert sind, dass die Gesamtmenge denaturierender Substanz zwischen 2 und 5 mg inklusive je Zusammensetzung ist.
